# EUROPÄISCHE PATENTANMELDUNG

(11) **EP 2 133 668 A1**
(43) Veröffentlichungstag der Anmeldung: **16.12.2009**
(21) Anmeldenummer: 08010702.2
(22) Anmeldetag: 12.06.2008
(51) Int. Cl.: G01F 22/00, G01F 23/292, G01B 11/02

(54) **Zerstörungsfreie Messung des Füllvolumens eines mit einer Flüssigkeit gefüllten Behälters**

(71) Anmelder: CSL Behring GmbH, 35041 Marburg (DE)
(72) Erfinder: Hoberg, Karl, 35043 Marburg (DE)

(57) **Zusammenfassung**

Die Erfindung schlägt ein Verfahren zur Bestimmung des Füllvolumens eines mit einer Flüssigkeit (30) zumindest teilweise gefüllten, verschlossenen Behälters (16) vor, wobei der Behälter mit elektromagnetischer Strahlung (26) beaufschlagt wird, wobei zumindest ein Teil der vom Behälter transmittierten Strahlung (28) in einer Bildebene ortsaufgelöst detektiert und einer Auswerteeinheit (22) zugeführt wird, wobei zur Füllvolumenbestimmung zumindest die Füllstandshöhe (36) der Flüssigkeit (30) im Behälter (16) und zumindest eine Innenquerschnittsfläche des Behälters (16) aus den detektierten Bilddaten ermittelt werden.

Die Erfindung schlägt ferner eine Vorrichtung zur Bestimmung des Füllvolumens eines mit einer Flüssigkeit zumindest teilweise gefüllten, verschlossenen Behälters sowie ein Computerprogrammprodukt mit Programmmitteln zur Bestimmung des Füllvolumens vor.

## Beschreibung

Die Erfindung betrifft ein Verfahren und eine Vorrichtung zur Bestimmung des Füllvolumens eines mit einer Flüssigkeit zumindest teilweise gefüllten, verschlossenen Behälters, insbesondere von Spritzen, Vials und Ampullen, welche mit einem flüssigen Produkt, wie etwa einem Impfstoff, befüllbar sind.

### Stand der Technik

In Rahmen der industriellen Massenfertigung von Medikamenten, insbesondere von mit flüssigen Wirkstoffen zu füllenden Glasbehältnissen ist es erforderlich, zur Prozesskontrolle und zur Steuerung einzelner Prozessparameter, die Füllmenge, bzw. das Volumen oder das Gewicht eines flüssigen Wirkstoffs zu möglichst präzise zu ermitteln. Die Messung oder Überprüfung der tatsächlichen Menge einer in einem geschlossenen Behälter abgefüllten Flüssigkeit oder die Bestimmung des Füllvolumens des Behälters, welcher z.B. ein flüssiges Medikament, etwa einen Impfstoff enthält, erfolgt typischerweise anhand einzelner Stichproben einer in Massenproduktion hergestellten und maschinell abgefüllten Charge, welche oftmals deutlich mehr als 100 einzelne gefüllte Behälter aufweisen kann.

Die stichprobenartige Messung eines zumindest bereichsweise gefüllten verschlossenen Behälters, der z.B. als Spritze, Flakon, Vial oder als Ampulle ausgebildet sein kann, erfolgt dadurch, dass mit einzelnen Behältern eine Brutto-Netto-Wägung durchgeführt wird. So wird der Behälter nacheinander, einmal mit dem darin befindlichen Produkt und einmal ohne das Medikament gewogen. Der Behälter selbst und das darin befindliche Medikament werden dabei jedoch zerstört. Durch eine solche Volumen- und Massenbestimmung gehen stets einige Behälter pro Charge verloren. Dies kann sich je nach Produkt und Chargengröße auf einen Verlust von 50 bis zu 200 Einheiten aufsummieren.

Die US 5,568,262 beschreibt ein zerstörungsfreies Messverfahren für einen verschlossenen und transparenten Behälter. Dabei ist vorgesehen, den Behälter mit hoher Geschwindigkeit um eine horizontale Achse rotieren zu lassen, sodass sich die Flüssigkeit aufgrund der Zentrifugalkraft in den außen liegenden Bereichen des Behälters sammelt und sich im mittleren Bereich des Behälters eine statische Luftblase bildet. Die Größe dieser Luftblase wird mittels einer Kamera und elektronischen bildgebenden Mitteln erfasst und vom zuvor anderweitig bestimmten Volumen des transparenten Behälters subtrahiert.

Hierzu muss unter anderem der Innendurchmesser des Behälters ermittelt werden, wofür ein Laser-Scanning-Verfahren vorgeschlagen wird, bei welchem eine Laserlichtquelle mittels eines so genannten Scanning Mirrors nacheinander auf einzelne Punkte des Behälters gerichtet wird. Ein Silhouettenabbild des Behälters wird dabei auf einem opaken, bzw. matten Schirm abgebildet und mittels einer Videokamera aufgenommen.

Obwohl schon diese Art der Volumenfüllstandskontrolle eine zerstörungsfreie Messung des Füllvolumens des Behälters ermöglicht, ist seine Implementierung doch recht komplex und aufwändig. So muss dabei insbesondere gewährleistet werden, dass die Rotationsachse des Behälters exakt mit der optischen Achse des optischen Messsystems übereinstimmt. Hierzu ist eine mitunter aufwändige Positionierung vom zu vermessenden Objekt und der Messoptik erforderlich. Ferner ist ein Laser-Scanning-Verfahren zur Ermittlung der Behältergeometrien aufgrund der dafür erforderlichen Komponenten relativ kostenintensiv in der Anschaffung.

Des Weiteren muss bei einem solchen Abtastverfahren gewährleistet werden, dass die zu vermessenden Proben über den gesamten Zeitraum der Abtastung keinen störenden Einflüssen, wie etwa mechanischen Erschütterungen unterliegen. Auch ist für ein solches Abtast-Verfahren stets ein gewisses Zeitintervall, oftmals im Bereich einiger Sekunden wenn nicht gar Minuten, erforderlich, um die gesamte Behältergeometrie mit hinreichender Genauigkeit ermitteln zu können.

Eine Lasereinstrahlung in dieser Zeitdauer kann bei pharmazeutischen Produkten zur Änderung der spezifizierten Eigenschaften führen, deshalb muss für jedes Produkt im Rahmen einer Validierung der Ausschluss negativer Einwirkung des Laserverfahrens nachgewiesen werden.

### Aufgabe

Der vorliegenden Erfindung liegt daher die Aufgabe zugrunde, ein vereinfachtes und zugleich verbessertes Verfahren zur Bestimmung des Füllvolumens eines mit einer Flüssigkeit gefüllten verschlossenen Behälters zur Verfügung zu stellen. Dieses soll eine zerstörungsfreie Messung des Füllvolumens mit verhältnismäßig einfachen und kostengünstig zu implementierenden Mitteln ermöglichen. Zudem soll die Messgenauigkeit gegenüber einer Brutto-Netto-Wägung verbessert werden. Auch ist es Ziel der Erfindung, eine Füllvolumenbestimmung äußerst präzise und mit einem deutlich verringerten Zeitaufwand durchzuführen.

### Erfindung und vorteilhafte Wirkungen

Die der Erfindung zugrunde liegende Aufgabe wird mittels eines Verfahrens gemäß Patentanspruch1 und mittels einer Vorrichtung gemäß Patentanspruch 12 gelöst. Weitere vorteilhafte Ausführungen der Erfindung sind in den zugehörigen Unteransprüchen angegeben.

Das erfindungsgemäße Verfahren zeichnet sich durch eine rein optische Messung und eine rein optische Ermittlung des Füllvolumens eines Behälters aus, der für die bei der Messung verwendete Strahlung im wesentlichen transparent ist. Bei dem vorzugsweise verschlossenen Behälter handelt es sich insbesondere um Spritzen, Vials und Ampullen, welche mit einem flüssigen Produkt, wie etwa einem Impfstoff, zumindest teilweise befüllt sind. Dabei ist vorgesehen, den Behälter einem rein optischen Prüfsystem zuzuführen und gegenüber einem Bild erzeugenden Messsystem zu positionieren, unter Verwendung geeigneter elektromagnetischer Strahlung zu beleuchten und mittels zumindest einem ortsauflösenden, das heißt in der Ebene transversal zur optischen Achse zweidimensional ausgebildeten Detektor entsprechende Bilddaten aufzunehmen und diese schließlich einer Auswerteeinheit zuzuführen.

Die geometrische Vermessung des Behälters und der im Behälter befindlichen Flüssigkeit erfolgt anhand der aufgenommenen zweidimensionalen Abbildungen des gefüllten Behälters. Dazu ist ein Rotieren des Behälters grundsätzlich nicht erforderlich. So ermöglicht die Erfindung die Durchführung einer vollständigen Füllvolumenmessung bereits anhand einer einzigen Aufnahme.

Die optische Vermessung beziehungsweise das Aufnehmen von Abbildungen des gefüllten Behälters erfolgt in Transmissionsgeometrie. Das heißt eine Strahlungs-oder Lichtquelle und ein zugehöriger Detektor werden so zueinander angeordnet, dass sich der zu vermessende Behälter zwischen Detektor und Strahlungsquelle befindet. Zumindest ist der Detektor derart angeordnet und ausgelegt, dass er zumindest einen Teil der vom Behälter transmittierten elektromagnetischen Strahlung in einer Bildebene ortsaufgelöst aufzeichnen kann.

Erfindungsgemäß ist vorgesehen, dass die Füllvolumenbestimmung der Flüssigkeit innerhalb des vorzugsweise geschlossenen Behälters unmittelbar aus den gewonnenen Bilddaten ermittelt wird. Hierzu werden zumindest zwei Parameter, nämlich die Füllstandshöhe der Flüssigkeit im Behälter und eine im Bereich der Füllstandshöhe liegende Innenquerschnittsfläche des Behälters unmittelbar aus den detektierten Bilddaten ermittelt. Das Füllvolumen ergibt sich dann als Produkt aus Füllstandshöhe und ermittelter Innenquerschnittsfläche.

Die Innenquerschnittsfläche wird aus der dem Messverfahren zugrunde liegenden und gegebenenfalls von einem Benutzer angegebenen Grundgeometrie des Behälters und des optisch ermittelbaren Innenquerschnitts des Behälters ermittelt. Weist der Behälter zum Beispiel eine zylindrische Geometrie auf, so wird dessen Innendurchmesser D als auch die Füllstandshöhe h aus den gewonnenen Bilddaten abgeleitet. Die Bestimmung des Füllvolumens V erfolgt nach der Formel: V=π D ² /4 Bei einem im Querschnitt quadratisch oder rechteckig ausgebildeten Behälter genügt es hingegen, die lichte Weite, beziehungsweise die Breite des Behälters aus den aufgenommenen Bilddaten zu ermitteln.

Als Strahlungsquelle kommen für das erfindungsgemäße Verfahren unterschiedlichste Lichtquellen mit einem Spektralbereich vom Infraroten bis zum Ultravioletten in Betracht. Die verwendete elektromagnetische Strahlung kann durch Verwendung entsprechender Filter monochromatisch sein oder aber ein breites Spektrum an unterschiedlichen Wellenlängen aufweisen. Grundsätzlich kann die gesamte optische Messung mittels Weißlicht durchgeführt werden.

Das erfindungsgemäße Verfahren ist insbesondere zur Füllvolumenbestimmung zylindrischer Behälter ausbildet, wobei die Zylinderlängsachse senkrecht zur optischen Achse des Messsystems angeordnet wird. Zur Ermittlung der Innenquerschnittsfläche des Behälters ist insbesondere vorgesehen, den Außendurchmesser des Behälters und dessen Wanddicke unmittelbar aus den aufgenommenen Bilddaten zu ermitteln.

Die Ermittlung der Wanddicke des Behälters erfolgt vorzugsweise durch eine telezentrische Ausleuchtung des Behälters. Dabei wird der Behälter mit einem Bündel paralleler Lichtstrahlen beaufschlagt, welche mittels einer objektseitigen Optik erzeugt und zumindest mittels einer bildseitigen telezentrischen Optik auf dem Detektor abgebildet werden. Bei der Beleuchtung eines transparenten zylindrischen Behälters mit parallelem Licht existieren aufgrund der Krümmung des Behälters nur einige wenige ausgezeichnete Stellen, an denen die Eintritts- und Austrittswinkel der parallelen Strahlen zueinander identisch sind, sodass ein in den Behälter eintretender parallel zur Mittelachse propagierenden Lichtstrahl im Wesentlichen ohne Richtungsänderung aus dem Behälter wieder austritt.

Diese Bedingungen werden vom Mittelpunktstrahl und von einigen durch die Behälterwandung propagierenden Strahlen erfüllt. Diese werden bei Eintritt in die Behälterwand gebeugt und an den Grenzflächen zum jeweils optisch dünneren Medium, das heißt an den Grenzflächen zum Behälterinnen- und -außenraum reflektiert. Bei einer telezentrischen Anordnung beziehungsweise bei einer telezentrischen Ausleuchtung des Objekts entstehen auf dem Detektor einzelne Intensitätsmaxima, welche den ein-, zwei-, vierfach reflektierten parallel zur optischen Achse durch das Objekt propagierenden Strahlen entsprechen. Aus dem Abstand der einzelnen Intensitätsmaxima und in Kenntnis der Brechzahl des Behältermediums kann die Dicke der Behälterwand und/oder des Innendurchmesser des Behälters präzise bestimmt werden.

Die telezentrische Ausleuchtung ist insoweit von Vorteil, als dass lediglich die einzelnen Intensitätsmaxima oder Intensitätspeaks paralleler Strahlen detektiert werden, während solche Strahlen, die etwa aufgrund der gekrümmten Kontur des Gefäßes nicht mehr parallel zur optischen Achse verlaufen, nicht auf den Detektor gelangen.

Durch die Verwendung eines telezentrischen Objektivs bzw. durch die vorgeschlagene telezentrische Beleuchtung kann die Wanddicke bzw. der Innendurchmesser des Behälters nicht nur an einer einzigen Stelle, sondern über einen längserstreckten Bereich entlang der Zylinderlängsachse präzise ermittelt werden. Auf diese Art und Weise können sogar Abweichungen in der Wanddicke, im Innendurchmesser oder Abweichungen von einer exakten zylindrischen Form des Behälters präzise ermittelt werden und bei den nachfolgenden Berechungen des Füllvolumens berücksichtigt werden.

Ein weiterer Vorteil der telezentrischen Ausleuchtung des zu vermessenden Objekts besteht darin, dass die Breite eines ein zylindrisches Innenvolumen begrenzenden Stopfens exakt dem Außendurchmesser des transparenten, vorzugsweise aus Glas gefertigten Behälters entspricht. Auf diese Art und Weise kann besonders einfach und zugleich präzise der Außendurchmesser des Behälters ermittelt werden.

Nach einem weiteren Aspekt der Erfindung wird die Füllstandshöhe der im Behälter befindlichen Flüssigkeit anhand der Positionen eines oberen und eines unteren Flüssigkeitsspiegels aus den detektierten Bilddaten ermittelt. Die Füllstandshöhe ergibt sich dabei aus einer Subtraktion ermittelter Positionen des oberen und des unteren jeweils optisch ermittelten Flüssigkeitspegels oder -spiegels.

Zur Ermittlung Pegelstände der Flüssigkeit bzw. der Positionen der Flüssigkeitsspiegel wird der Behälter diffus be- oder durchleuchtet, sodass die Flüssigkeitsspiegel beziehungsweise der Außenumfang des Behälters unmittelbar auf dem Detektor abgebildet werden. Für diese diffuse Ausleuchtung oder Beleuchtung des Behälters kann eine breitbandige Weißlichtquelle ggf. in Kombination mit spektralen Filtern vorgesehen werden. Auch kann hierzu Licht im infraroten Wellenlängenbereich Verwendung finden. Die telezentrische Ausleuchtung des zu vermessenden Objektes und dessen diffuse Beleuchtung kann ferner auch aus einer einzigen Lichtquelle gespeist werden.

Daneben ist auch denkbar, die zur Bestimmung des Innendurchmessers vorgesehene telezentrische Ausleuchtung in einem anderen Spektralbereich durchzuführen, wie er zur Ermittlung der Füllstandshöhe vorgesehen ist. Unabhängig davon ist es denkbar, durch Verwendung zweier Detektor- oder Kamerasysteme sowohl eine diffuse als auch telezentrische Beleuchtung des Behälters simultan durchzuführen. Da bei der Verwendung eines telezentrischen Objektivs ohnehin nur parallel zur optischen Achse propagierende Strahlen auf dem Detektor abgebildet werden, stellt eine gleichzeitige diffuse Be- oder Ausleuchtung des Behälters eine kaum nennenswerte Störung der telezentrischen Beleuchtung und Ermittlung der Wanddicke dar.

Eine Positionsbestimmung zumindest eines Flüssigkeitsspiegels erfolgt durch zeilen- und/oder spaltenweises Vergleichen von Intensitäten einzelner Pixel des Detektors. Dabei kann ferner vorgesehen werden, nicht nur die gemessenen Intensitäten untereinander zeilen- und spaltenweise, sondern auch absolut, das heißt mit einem vorgegebenen Schwellwert zu vergleichen. Ein solcher Schwellwert kann insbesondere bei der Kalibrierung des Messsystems ermittelt und festgelegt werden.
Weiterhin kann die Relation der in diffuser Beleuchtung bestimmten Pixelzahl des Außendurchmessers zum telezentrisch gemessenen Außendurchmesser ermittelt werden. Dies ermöglicht eine Kalibrierung des Systems, nämlich die Zuordnung einer Pixelanzahl zu tatsächlichen Längenmaßen, sodass durch Ermittlung einer entsprechenden Pixelanzahl die tatsächliche Höhe der Flüssigkeitssäule bestimmt werden kann.

In diesem Zusammenhang ist es insbesondere von Vorteil, elektromagnetische Strahlung zu verwenden, für welche das Behältermaterial und/oder die im Behälter befindliche Flüssigkeit einen gegenüber Luft deutlich veränderten Transmissions-oder Absorptionskoeffizienten aufweist. Auf diese Art und Weise entstehen in den aufgenommenen Bildern deutliche Intensitätsunterschiede, die zu einer präzisen Ermittlung der zu bestimmenden Grenzflächen herangezogen werden können.

Nach einer vorteilhaften Weiterbildung der Erfindung ist ferner vorgesehen, für die Zuordnung von einer in der Bildebene vorhandenen Anzahl an Pixel zu einem tatsächlichen Längenmaß, den Pixelwert eines mittels diffuser Beleuchtung ermittelten Außendurchmessers des Behälters mit demjenigen Pixelwert zu vergleichen, welcher mittels telezentrischer Beleuchtung ermittelbar ist.

Dabei wird in vorteilhafter Weise der konstante Abbildungsmaßstab der telezentrischen Ausleuchtung ausgenutzt. So ist in einem ersten Schritt vorgesehen, mittels der telezentrischen Messanordnung die Größe eines charakteristischen Objekts, so etwa die Breite eines Spitzenstopfens einer auf dem Detektor entsprechenden Pixelanzahl zuzuordnen. Die telezentrische Optik ermöglicht dabei aufgrund der verwendeten parallelen Strahlen bereits eine exakte Zuordnung einer Pixelanzahl zu einer tatsächlichen Messgröße, etwa der Breite des Spritzenstopfens in mm.

In einer nachfolgenden Messung, welche mit diffuser Beleuchtung erfolgt kann der Größe desselben charakteristischen Objekts, vorzugsweise der Breite des Spritzenstopfen, erneut eine Pixelanzahl zugeordnet werden. Durch den Vergleich der mit den beiden unterschiedlichen optischen Messverfahren ermittelten Pixelanzahl ein und desselben Objekts und anhand der konstanten Skalierung der telezentrischen Optik kann eine besonders exakte Skalierung des Systems auch für die diffuse Beleuchtungsanordnung durchgeführt werden.

Hierdurch wird es insbesondere ermöglicht, für jedes einzelne Testobjekt, also für jede Spritze, eigens eine Skalierung der mittels diffuser Beleuchtung ermittelbaren Pixelzahl durchzuführen.

Bei der Vermessung der Flüssigkeitsspiegel und/oder des Außendurchmessers des Behälters ist die optische Achse des Messsystems senkrecht zur Zylinderachse des zylindrischen Behälters ausgerichtet. Dabei kann der obere und/oder untere Flüssigkeitsspiegel entweder als gerader Strich oder als Oval auf dem Detektor abgebildet werden. Sofern sich einer der Flüssigkeitsspiegel als Oval darstellt, werden die gekrümmten Außenkonturen des Flüssigkeitsspiegels aus den gewonnenen Bilddaten ermittelt und einer geometrische Mittelung in der nachgeschalteten Auswerteeinheit unterzogen, sodass anstelle des tatsächlich gemessenen ovalen Flüssigkeitsspiegels ein gemittelter geradlinig verlaufender Flüssigkeitsspiegel zur Bestimmung der Füllstandshöhe verwendet werden kann.

Vorzugsweise wird der Behälter, insbesondere eine Spritze mit einer nach oben gerichteten Spitze im Messsystem angeordnet, sodass die zur Flüssigkeit hin gerichtete Grenzfläche eines das Behälterinnenvolumen begrenzenden Stopfens zugleich dem unteren Flüssigkeitsspiegel entspricht. Der Stopfen hat vorzugsweise eine stark unterschiedliche Transmissionscharakteristik für die verwendete Strahlung als der Wirkstoff und die Behälterwand. Er weist zudem eine dunkle Farbe, vorzugsweise schwarz auf, damit die gewonnenen Bilddaten besonders kontrastreich sind.

Der im Behälter eingeschlossene Wirkstoff hat vorzugsweise gegenüber dem Behältermaterial eine unterschiedliche Transmissionscharakteristik, sodass an der Grenzfläche zwischen den Stoffen und der Flüssigkeit beziehungsweise der Behälteraußenwand deutliche Intensitätsunterschiede in den aufgenommenen Bilddaten erkennbar sind.

Die Position des Stopfens kann dadurch ermittelt werden, dass das aufgenommene Bild zeilenweise in einer Richtung senkrecht zur Stopfenoberfläche abgetastet und analysiert wird. Dabei wird jede Zeile als Stopfenposition angenommen, welche die größte Grauwertänderung gegenüber einer benachbarten Zeile aufweist. Auf diese Art und Weise kann z.B. ein womöglich störender Einfluss von am Stopfen anhaftenden Luftblasen eliminiert werden. Ferner kann die Quererstreckung, das heißt der Durchmesser des vorzugsweise nicht transparenten Stopfens ermittelt werden.

Die Positionen der Flüssigkeitsspiegel sowie die Abmessungen des Stopfens werden als Pixel in der dem Detektor nachgeschalteten Auswerteeinheit ermittelt. Die Auswerteeinheit vergleicht die Pixelzahl des so ermittelten Außendurchmessers mit dem über telezentrische Optik gemessenen Außendurchmessers. Über dieses Verhältnis wird die Pixelzahl der Füllstandshöhe in ein tatsächliches Längenmaß umgerechnet.

Dabei werden die der Füllstandshöhe als auch dem Durchmesser des Behälters entsprechende Anzahl an Pixel bestimmt, welche mittels einer zuvor vorgenommenen Kalibrierung in geometrische und tatsächliche Längenmaße und Volumina umgerechnet werden.

Weiterhin ist es im Rahmen der Erfindung vorgesehen, dass eine Vorrichtung zur Konzentration der Flüssigkeit im zylindrischen Teil des Behältnisses vor Beginn der Messung eingesetzt wird. Bei Spritzen wird vorzugsweise eine Rotation um die Längsachse des Behältnisses angewendet, um Flüssigkeitsreste aus der Spitze in den Zylinder zu transportieren. Die Rotationsachse ist hierbei vorzugsweise senkrecht, zumindest aber schräg zur optischen Achse des Detektors beziehungsweise des optischen Aufnahmesystems ausgerichtet. Bei anderen Behältnissen, wie z.B. Ampullen, kann die Anwendung einer mechanischen Schwingvorrichtung oder eine Ultraschallbehandlung vorgesehen werden.

Weiterhin ist es im Rahmen der Erfindung vorgesehen, dass zur Eliminierung oder zur Umverteilung von innerhalb der Flüssigkeit vorhandenen Gasblasen, insbesondere von Luftblasen, der Behälter zumindest zeitweise in Rotation, vorzugsweise um die Zylinderlängsachse, versetzt wird. Das heißt die Rotationsachse ist dabei senkrecht, zumindest aber schräg zur optischen Achse des Detektors beziehungsweise des optischen Aufnahmesystems ausgerichtet.

Durch die zumindest zeitweise Rotationsbewegung des gesamten Behälters kommt es zu einer Umverteilung von in der Flüssigkeit eingeschlossenen und/oder an den Gefäßwänden anhaftenden Luftbläschen. Diese wandern infolge der Bewegung des gesamten Behälters an die Oberfläche der Flüssigkeit beziehungsweise hin zur Grenzfläche von Luft und Flüssigkeit.

Nach einer alternativen Ausführung der Erfindung kann vorgesehen sein, die in der Flüssigkeit eingeschlossenen Gas- oder Luftblasen ihrer Größe nach aus den zu ermitteln, indem deren maximaler Querschnitt aus den aufgenommenen Bilddaten ermittelt und entsprechend der Kreisvolumenformel in ein Gasblasenvolumen umgerechnet wird, welches von dem ermittelbaren Füllstandsvolumen subtrahiert werden kann. Bei der Bestimmung der Volumina von Luftblasen wird zudem die Annahme getroffen, dass diejenigen Gasblasen, welche sowohl an die Zylinderseitenwand als auch an eine Stirnwand angrenzen, näherungsweise eine Viertelkugel bilden, während solche Gasblasen, die vollständig an die Zylinderinnenwand angrenzen näherungsweise als Halbkugel angenommen werden können.

Es hat sich in der Praxis ferner als sinnvoll erwiesen, nur solche Gasblasen bei der Füllvolumenbestimmung zu berücksichtigen, deren Durchmesser oberhalb eines vorgegebenen Schwellwertes liegt. Es hat sich als vorteilhaft erwiesen, nur solche Gasbläschen zur berücksichtigen, deren Durchmesser größer als 1 mm, vorzugsweise größer als 2 mm ist.

Nach einer weiteren Ausführungsform ist vorgesehen, dass von ein und demselben Behälter eine Abfolge von n Bilddaten zeitlich versetzt aufgenommen wird, wobei der Behälter zwischen den einzelnen Aufnahmen um jeweils einen n-tel Vollkreis um eine Achse senkrecht zur optischen Achse, insbesondere um die Zylinderlängsachse gedreht wird. Durch einen Vergleich oder durch eine Mittelung der aufgenommenen Bildsequenz können Unregelmäßigkeiten in der Geometrie des Behälters und in der Flüssigkeitsverteilung, beispielsweise durch eine einseitige Anhaftung von Flüssigkeit aufgrund eines Oberflächenspannungseffektes erkannt und bei der Volumenbestimmung berücksichtigt werden.

Auch wird durch den Vergleich der insgesamt n Bilddaten untereinander ermöglicht, einen maximalen Querschnitt oder Durchmesser eingeschlossener Luftblasen zu ermitteln.

Nach einem weiteren unabhängigen Aspekt betrifft die Erfindung eine Vorrichtung zur Bestimmung des Volumens eines mit einer Flüssigkeit zumindest teilweise gefüllten Behälters. Die Vorrichtung weist zumindest eine Strahlungsquelle zur Emission elektromagnetischer Strahlung und einen mit einer Auswerteeinheit koppelbaren Detektor, insbesondere eine CCD- oder Videokamera zur Aufnahme von zweidimensionalen Bilddaten des zwischen Strahlungsquelle und Detektor angeordneten Behälters auf. Die Vorrichtung ist insbesondere zur Aufnahme von Bilddaten des Behälters in Transmissionsgeometrie ausgelegt, sodass die durch den Behälter und die darin enthaltene Flüssigkeit transmittierte Strahlung vom Detektor aufgenommen und der nachgeschalteten Auswerteeinheit zur Bildauswertung zugeführt werden kann.

Zur Aufnahme scharfer und skalierter Bilddaten sind refraktive und/oder diffraktive Strahlführungsmittel zwischen dem Detektor und der Strahlungsquelle vorgesehen. Diese weisen einzelne Abbildungslinsen, vorzugsweise zumindest ein für die Abbildung des Behälters auf dem Detektor ausgebildetes Objektiv auf. Die dem Detektor nachgeschaltete Auswerteeinheit ist zur Bestimmung des Füllvolumens, also der Flüssigkeitsmenge im Behälter ausgebildet. Dabei werden aus den Bilddaten eine Füllstandshöhe der Flüssigkeit im vorzugsweise zylindrischen Behälter und zumindest der Innenquerschnitt des Behälters, beziehungsweise eine Innenquerschnittsfläche selbsttätig ermittelt.

Die Vorrichtung zeichnet sich vor allem dadurch aus, dass zur Bestimmung des Innenquerschnitts des Behälters eine Beaufschlagung des Behälters mit einem parallelen Strahlenbündel vorgesehen und zumindest ein telezentrisches Objektiv zwischen Detektor und Strahlungsquelle angeordnet ist. Die telezentrische Optik kann dabei als objektseitiges und/oder bildseitiges Objektiv vorgesehen werden. Mittels der telezentrischen Ausleuchtung des Behälters kann in einfacher und präziser Art und Weise der Innendurchmesser des Behälters ermittelt und unmittelbar zur Bestimmung des Innenquerschnitts verwendet werden.

Weiterhin ist ein um eine Drehachse drehbar gelagerter Teller zur Aufnahme des Behälters vorgesehen. Die Anordnung des Behälters an dem Drehteller ist dergestalt, dass die Zylinderlängsachse des Behälters und die Drehachse des Drehtellers zueinander parallel ausgerichtet sind oder gar zusammenfallen.

Nach einem weiteren unabhängigen Aspekt umfasst die Erfindung ein Computerprogrammprodukt mit Programmmitteln zur Bestimmung des Füllvolumens eines mit einer Flüssigkeit zumindest teilweise gefüllten Behälters, welche dazu ausgebildet sind, aus den von einem Detektor zur Verfügung gestellten Bilddaten eine Füllstandshöhe der Flüssigkeit und einen Innendurchmesser des Behälters nach dem oben beschriebenen Verfahren selbsttätig zu ermitteln und zur Bestimmung des Füllstandsvolumens miteinander zu multiplizieren.

Weitere Aspekte sowie Merkmale und vorteilhafte Anwendungsmöglichkeiten der Erfindung sind in der nachfolgenden Beschreibung der Figuren selbst dargestellt. Dabei ist anzumerken, dass sämtliche Einzelmerkmale in jeglicher sinnvollen Kombination den Gegenstand der vorliegenden Erfindung bilden. Es zeigen:
- Figur 1: eine schematische Darstellung einer rein optischen Messeinrichtung zur Ermittlung des Füllstandsvolumens eines mit Flüssigkeit gefüllten Behälters,
- Figur 2: eine schematische Darstellung eines als Spritze oder Ampulle ausgebildeten Behälters mit einer nach unten gerichteten Spitze,
- Figur 3: die Spritze gemäß Figur 2 mit einer nach oben gerichteten Spitze,
- Figur 4: eine vergrößerte Darstellung eines Behälterbereichs im Bereich des Flüssigkeitsvolumens,
- Figur 5: eine schematische Darstellung eines zylindrischen Behälters im Querschnitt bei telezentrischer Ausleuchtung,
- Figur 6: ein schematisches Intensitätsprofil einer typischen Intensitätsverteilung bei telezentrischer Ausleuchtung gemäß Figur 5,
- Figur 7: eine schematische zweidimensionale Intensitätsverteilung bei telezentrischer Ausleuchtung über den gesamten Bereich der Flüssigkeitssäule,
- Figur 8: eine schematische Darstellung eines mit Flüssigkeit teilweise gefüllten Behälters und
- Figur 9: eine schematische Darstellung einer Gasblase inmitten der Flüssigkeit.

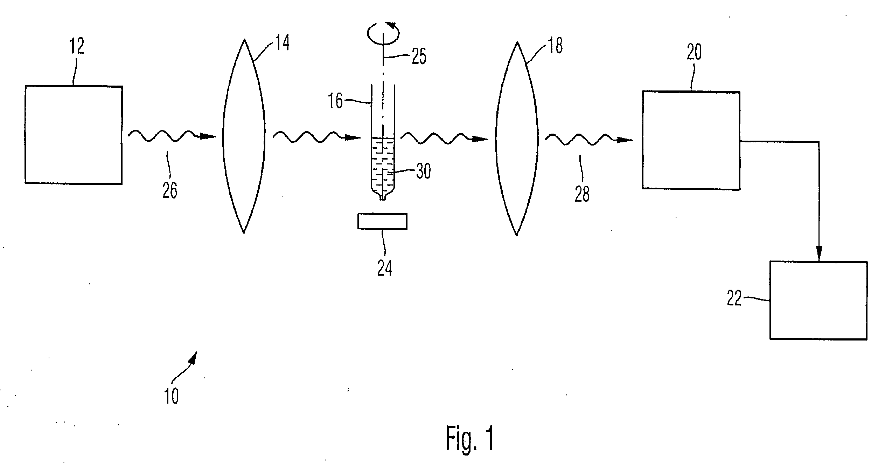

Das in Figur 1 schematisch wiedergegebene Messsystem 10 ist zur rein optischen Füllvolumenermittlung eines mit einer Flüssigkeit 30 zumindest teilweise gefüllten Behälters 16 ausgebildet. Es weist eine Strahlungsquelle 12 elektromagnetischer Strahlung 26 sowie einen zur Aufnahme und Detektion transmittierter elektromagnetischer Strahlung 28 vorgesehenen Detektor 20 auf. Dem Detektor, welcher typischerweise als CCD-Kamera ausgebildet ist, ist eine Bildauswerteeinheit 22 nachgeschaltet, welche aus den ermittelten und vom Detektor aufgenommenen Bilddaten selbsttätig sowohl die Füllstandshöhe der Flüssigkeit 30 als auch den Innendurchmesser des Behälters 25 ermitteln kann.

Der Behälter 16, welcher typischerweise als Spritze, Ampulle oder Vial, das heißt als Glasröhrchen, ausgebildet sein kann, wird auf einem drehbar gelagerten Drehteller 24 angeordnet. Der Drehteller 24 ermöglicht die Rotation des Behälters 16 um seine Zylinderlängsachse 25, um von ein und demselben Behälter eine Sequenz von Bilddaten zu den unterschiedlichen Orientierungen des Behälters 16 aufnehmen zu können. Der Behälter ist vorzugsweise aus transparentem Glas gefertigt. Andere Ausgestaltungen, sowie die Verwendung von diversen transparenten Kunststoffen als Behältermaterial kommen jedoch ebenso infrage.

Zwischen dem Behälter 16 und der Kamera 20 ist eine Abbildungsoptik 18 vorgesehen, welche auf der Detektorfläche der Kamera 20 ein scharfes Abbild des aus Glas gefertigten Behälters 16 zur Verfügung stellt. Optional kann auch zwischen der Strahlungsquelle 12 und dem Behälter 16 eine strahlformende Optik 14 angeordnet werden.

Die optische Vermessung des Behälters 16 und der darin befindlichen Flüssigkeit 30 erfolgt statisch, das heißt der Behälter 16 befindet sich in einem Ruhezustand gegenüber der Kamera 20.

Als Lichtquelle 12 kommt beispielsweise eine Weißlichtquelle oder eine Gasentladungslampe zum Einsatz. Es können auch Laserlichtquellen im gesamten sichtbaren, infraroten und ultravioletten Spektralbereich verwendet werden. Die Wellenlänge der verwendeten Strahlung 26 ist insbesondere an die optischen Transmissions-und Absorptionseigenschaften des Behälters 16 und der darin befindlichen Flüssigkeit 30 abgestimmt. So ist es beispielsweise von Vorteil, wenn die Behälterwand und die darin befindliche Flüssigkeit unterschiedliche Transmissionskoeffizienten für die verwendete Strahlung 26 aufweisen.

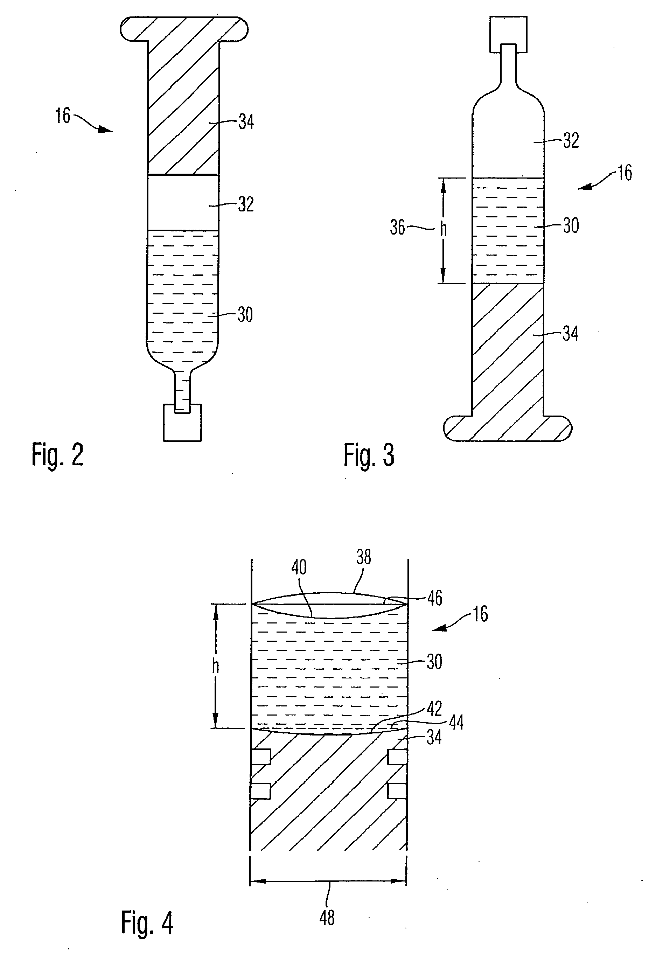

Grundsätzlich kann eine Ampulle oder Spritze 16, wie in den Figuren 2 oder 3 gezeigt, zur erfindungsgemäßen optischen Vermessung angeordnet werden. Bei der Darstellung gemäß Figur 2 zeigt die Spitze der Ampulle oder der Spritze 16 nach unten, während in Figur 3 die Spritze 16 mit ihrer Spitze nach oben zeigt. In der Konfiguration gemäß Figur 3 ist die in der zylindrischen Spritze 16 befindliche Flüssigkeit 30 nach unten von einem als Verschluss dienenden Stopfen 34 und nach oben von einem Luftvolumen 32 begrenzt. Zur Ermittlung der Füllstandshöhe 36 (h) ist es daher erforderlich, den oberen Flüssigkeitspegel oder Flüssigkeitsspiegel 46 und den unteren Flüssigkeitspegel oder -spiegel 44 präzise zu ermitteln, um durch Subtraktion der gemessenen Positionen die Füllstandshöhe h berechnen zu können.

Die Bestimmung der beiden Flüssigkeitsspiegel 46, 44 erfolgt mit einer Beleuchtung des Behälters 16 mit diffusem Licht, sodass für die Beaufschlagung des Behälters 16 mit elektromagnetischer Strahlung eine gerichtete und kollimierte Strahlungsquelle nicht erforderlich ist. Auch können die Pegelstände 46, 44 als auch der Außendurchmesser 48 des Behälters 16 in Reflektionsgeometrie präzise ermittelt werden.

Der Außendurchmesser des Behälters kann sowohl in diffuser aber auch mittels der telezentrischen Ausleuchtung ermittelt werden. Bei telezentrischer Ausleuchtung kann zudem der vorteilhafte Effekt ausgenutzt werden, dass die Breite des an der Behälterinnenwand angrenzenden Stopfens exakt der Breite bzw. dem Außendurchmesser des Glasbehälters entspricht.

Die selbsttätige Bestimmung der Flüssigkeitsspiegel 46, 44 als auch die Bestimmung des Außendurchmessers 48 des Behälters 16 erfolgt durch Abgleich der Pixelzahl des Außendurchmessers 48 mit dem tatsächlichen Längenmaß des Außendurchmessers 58. Die optische Vermessung der Positionen der Flüssigkeitsspiegel 46, 44 erfolgt in Durchlicht, also in Transmissionsgeometrie. Dazu wird eine großflächige diffuse Hintergrundbeleuchtung in Kombination mit einem Objektiv, beispielsweise einem 40 mm Objektiv, eingesetzt.

Es ist ferner vorgesehen, die Kamera 20 um 90 Grad um die optische Achse gedreht anzuordnen, damit die längere Seite des rechteckigen Detektors parallel zur Längsachse des zylindrischen Behälters 16 zu liegen kommt. Auch ist vorgesehen, die optische Achse der Kamera senkrecht zur Längsachse des Behälters 16 auszurichten. Dadurch kann der obere und/oder der untere Flüssigkeitsspiegel 46, 44, wie in Figur 4 angedeutet, entweder als gerader Strich oder leicht elliptisch auf der Kamera abgebildet werden. Ob es sich um eine ovale oder elliptische Darstellung handelt, wird anhand des entlang der Zylinderlängsachse verlaufenden Intensitätsprofils in der Behältermitte geprüft.

Sollte diese Prüfung die Existenz zweier gekrümmter Außenkonturen 38, 40 ergeben, so wird deren Verlauf aus den gewonnenen Bilddaten mittels eines Konturenverfolgungsverfahrens ermittelt. Anschließend wird anhand der ermittelten Außenkonturen 38, 40 ein idealisierter Flüssigkeitsspiegel 46 gemittelt oder berechnet.

Ähnlich wird die Position des Stopfens 34 ermittelt, welcher zugleich einen unteren Flüssigkeitsspiegel 44 definiert. Der mit radialen Rippen versehene Stopfen 34 ist vorzugsweise aus einem für die Strahlung 26 nicht transparenten Material gefertigt. Die Ermittlung des unteren Flüssigkeitsspiegels 44 kann durch ein sukzessives vertikales Abfahren einer horizontalen Linie erfolgen. Eine maximale Grauwert- oder Intensitätsänderung in Abtastrichtung parallel zur Zylinderlängsachse legt die Position 44 des unteren Flüssigkeitsspiegels fest. Die Füllstandshöhe h 36 kann durch Subtraktion der Positionen von oberem Flüssigkeitsspiegel 46 und unterem Flüssigkeitsspiegel 44 ermittelt werden. Natürlich liegen den aufgenommenen Bilddaten eine Kalibrierung und ein Umrechnungsfaktor in tatsächliche Größen- und Längenmaße zugrunde.

Ähnlich wie die beiden oberen Grenzlinien 38, 40, welche zum oberen Flüssigkeitsspiegel 46 gemittelt werden, kann es sich bei der unteren Grenzlinie 42 ebenfalls um eine gekrümmte Darstellung handeln. Durch Abtasten bzw. Abscannen einer in sich Figur 4 horizontalen erstreckenden Bildzeile in vertikaler Richtung kann jedoch auch hier ein geradliniger unterer Flüssigkeitsspiegel 44 berechnet und substituiert werden.

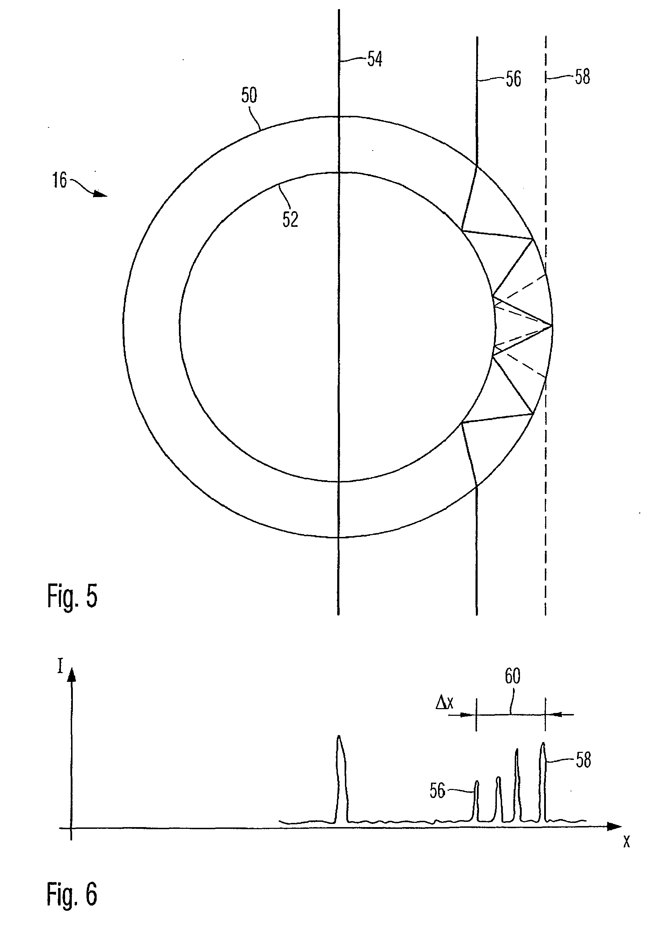

Der Außendurchmesser 48 des Behälters 16 wird anhand der radialen Ausdehnung des Stopfens 34 im Bereich zur Grenzlinie 42 bestimmt. Die Ermittlung der radialen Ausdehnung des Stopfens 34 und somit des Behälters 16 kann sowohl mittels der diffusen Ausleuchtung des Behälters 16, aber auch in der beschriebenen telezentrischen Konfiguration erfolgen, das heißt mithilfe einem Bündel parallel zur optischen Achse propagierender Strahlen. Durch Verwendung eines telezentrischen Objektivs 18 gelangen nur parallel zur optischen Achse propagierende Strahlen 28 auf den Detektor der Kamera 20. Diese Strahlpropagation durch den zylindrischen Behälter 16 ist schematisch in Figur 5 skizziert.

So propagiert lediglich der Mittelpunktstrahl 54 ungebrochen durch die Zylinderau-ßenwand 50 und die Zylinderinnenwand 52, während die mit einem seitlichen Versatz zum Mittelpunktstrahl 54 propagierenden Strahlen 56 und 58 an den Grenzflächen 50, 52 eine mehrfache Brechung beziehungsweise Reflexion erfahren. Die Strahlpropagation der beiden randseitig exemplarisch dargestellten parallelen Strahlen 56, 58 verläuft symmetrisch zu einer nicht explizit gezeigten horizontalen Mittelpunktachse des zylindrischen Behälters 16.

Durch die in Figur 5 angedeutete telezentrische Durchleuchtung des Behälters 16 entsteht in der in Figur 6 im Querschnitt dargestellten Bildebene eine Intensitätsverteilung mit einzelnen Spitzen, deren Abstand zueinander ein direktes Maß für die Wanddicke des Behälters ist. Auch kann der Innendurchmesser des Behälters durch Ermittlung des Abstandes zweier Intensitätsmaxima gleicher Ordnung, welche in Figur 5 rechts und links vom Mittelpunktstrahl auf den Detektor treffen, direkt bestimmt werden. Sofern der Brechungsindex des Behältermaterials bekannt ist, kann aus den Abständen der Peaks 56 und 58 unmittelbar die Wanddicke und der Innendurchmesser des Behälters ermittelt werden. Zur Kalibrierung der Wanddickenmessung kann der Innendurchmesser beispielsweise mittels einer Innenmessschraube mechanisch abgetastet und ermittelt werden. Darüber hinaus wäre auch eine Kalibrierung durch eine Vergleichsmessung mit einer weiteren Wellenlänge möglich, da die Brechungswinkel eintretender Strahlen nicht nur von der Brechzahl des Mediums, sondern auch von der Wellenlänge der verwendeten Strahlung abhängen. Ist die mittels telezentrischer Ausleuchtung erhältliche Abbildung einmal kalibriert kann diese zur wiederholenden Kalibrierung der mittels diffuser Beleuchtung gewonnen Bilddaten verwendet werden.

In Figur 5 sind einzig zwei randseitige Strahlengänge 56, 58 exemplarisch dargestellt. Während der Strahlengang 58 insgesamt 3 Reflexionen erfährt, weist der Strahlengang 56 insgesamt 7 Reflexionen auf. Daneben existieren auch noch Strahlengänge mit 5 Reflexionen, die hier aber nicht explizit wiedergegeben sind. Selbstredend kann auch auf der in Figur 5 linken Seite eine ergänzende Wanddickemessung nach dem gleichen Prinzip durchgeführt werden. Auch können die ermittelten Wanddicken über mehrere Messungen des Zylinders, zwischen welchen dieser um seine Längsachse gedreht ist, gemittelt werden. Ferner ist denkbar, die links und rechts des Mittelpunktstrahls detektierbaren Intensitätspeaks zur direkten Ermittlung des Innendurchmessers des Behälters miteinander zu vergleichen.

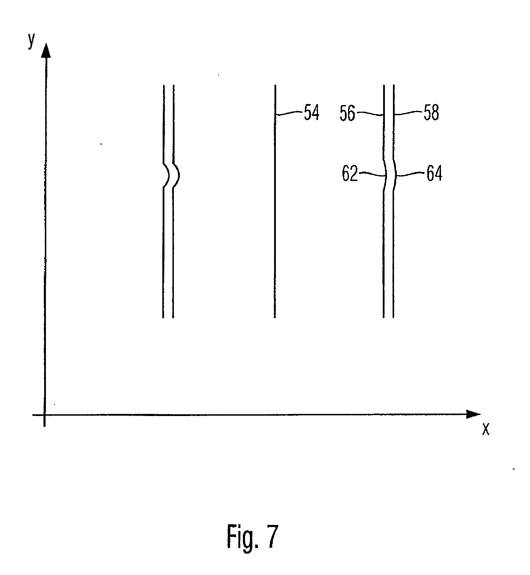

In Figur 7 ist schematisch ein sich bei der telezentrischen Durchleuchtung ergebendes zweidimensionales Intensitätsmuster dargestellt. Durch die flächenmäßige Durchleuchtung des Behälters 16 mit parallelen Strahlenbündeln können natürlich auch Unregelmäßigkeiten in der Geometrie der Behälterwand verhältnismäßig einfach und präzise ermittelt werden. So ist eine nach außen gekrümmte Wölbung einer Behälterwand 62, 64 unmittelbar in dem bei telezentrischer Konfiguration ermittelbaren Bild erkennbar.

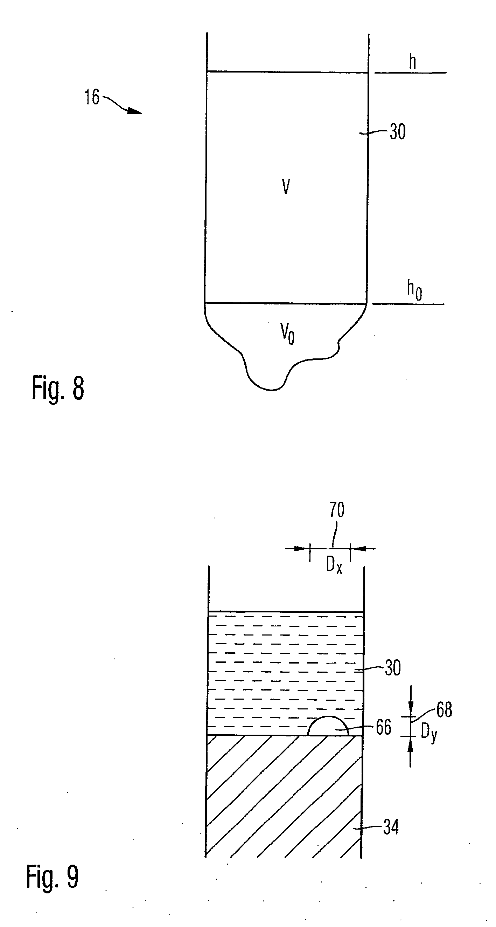

Figur 8 zeigt ferner eine Messkonfiguration, bei welcher beispielsweise eine Spritze 16 mit ihrer Spitze nach unten gerichtet auf dem Drehteller 24 angeordnet ist. Bei einer solchen Messung können zwar Höhenlinien h und h₀ zur Bestimmung eines Volumens V angenommen und vermessen werden. Das dabei ermittelte Volumen ist jedoch noch um das Volumen V₀ zu korrigieren. h₀ ist dabei ein frei definierbares Höhenmaß, das in die Nähe des Bereichs der Spritze 16 gelegt wird, bei der der zylindrische Bereich in den Spitzenbereich übergeht. Sollte sich in dem Bereich Vₒ keine Flüssigkeit befinden, so wären die in der Konfiguration nach Figur 8 und in der Konfiguration nach Figur 3 ermittelbaren Volumina identisch. Bei einer Referenzmessung, die nach der Konfiguration gemäß Figur 2 beziehungsweise gemäß Figur 8 durchgeführt wird, muss lediglich sichergestellt werden, dass sich in dem Volumenbereich Vₒ keine Flüssigkeit befindet. Aus einem Vergleich dieser Referenzmessung mit einer Messung, bei welcher der Bereich Vₒ vollständig mit Flüssigkeit gefüllt ist, lässt sich dann das Volumen Vₒ ermitteln, sodass bei der Annahme, dass sämtliche zu vermessenden Spritzen im Bereich der Spitze keine nennenswerten Abweichungen in ihrer Geometrie aufweisen, auch eine Messung mit nach unten gerichteter Spitze problemlos durchgeführt werden kann.

Figur 9 zeigt beispielhaft eine innerhalb der Flüssigkeit 30 befindliche Luftblase 66. Diese befindet sich vollständig an der Grenzfläche zwischen dem Stopfen 34 und der Flüssigkeit 30, sodass für deren Volumen eine Halbkugel angenommen werden kann. Hierzu werden der horizontale Durchmesser dx 70 und/oder der vertikale Durchmesser dy 68 ermittelt, gegebenenfalls gemittelt und zur Volumenberechnung der Luftblase 66 herangezogen.

Befände sich die Luftblase 66 sowohl an der Zylinderwandung als auch am Stopfen 34, würde man eine Viertelkugel für die Berechnung dem Volumen zugrunde legen.

In praktischen Versuchen hat sich gezeigt, dass der Flüssigkeitsspiegel, wie er in Figur 4 beispielhaft dargestellt ist, nicht immer eine Linie oder ein Oval darstellt, sondern dass sich aufgrund von inhärenten Oberflächenspannungen auch asymmetrische Anhaftungen von Flüssigkeit an der Behälterinnenwand ausbilden können. Solche durch Oberflächenspannungen verursachten Unregelmäßigkeiten können durch Vergleich mehrerer, unter verschiedenen Drehwinkeln des Drehtellers 24 aufgenommener Bilddaten gemittelt und korrigiert werden.

Zur Steigerung der Messgenauigkeit von sowohl der Wanddicke, der Innen- und Außendurchmesser als auch der Flüssigkeitspegel 44, 46 ist es nach der Erfindung insbesondere vorgesehen, den Drehteller 24 in insgesamt 8 verschiedenen Positionen mit einem Schrittmotor um jeweils 45 Grand zu drehen, um somit jeweils 8 Aufnahmen für jedes Testobjekt zu erhalten. Durch den Vergleich der gewonnenen Messdaten untereinander können somit Fehlerabweichungen auf ein Minimum reduziert werden.

Geht man von einer Füllvolumenmenge im Bereich von etwa 0,5 ml aus, so können Messgenauigkeiten von unter 10 Mikroliter (µl) erreicht werden. Der verbleibende Restfehler bei einer typischen Spritzengeometrie ist mit weniger als 5 µl hinsichtlich der Fehlerquellen Oberflächenspannung, Produkt in der Spritze, Abweichungen vom Innendurchmesser oder Fehler durch den gewählten Abbildungsmaßstab anzugeben. Hinsichtlich der Gasblaseneinschlüsse kann sogar ein Restfehler von unter 2 µl erreicht werden.

### Bezugszeichenliste

- 10: Messsystem
- 12: Strahlungsquelle
- 14: Objektiv
- 16: Behälter
- 18: Objektiv
- 20: Detektor
- 22: Auswerteeinheit
- 24: Drehteller
- 25: Drehachse
- 26: Strahlung
- 28: Transmittierte Strahlung
- 30: Flüssigkeit
- 32: Luft
- 34: Stopfen
- 36: Füllstandshöhe h
- 38: Grenzlinie
- 40: Grenzlinie
- 42: Grenzlinie
- 44: Unterer Flüssigkeitsspiegel
- 46: Oberer Flüssigkeitsspiegel
- 48: Außendurchmesser
- 50: Außenwand
- 52: Innenwand
- 54: Mittelpunktstrahl
- 56: Lichtstrahl
- 58: Lichtstrahl
- 60: Abstand
- 62: Wölbung
- 64: Wölbung

- 66: Gasblase
- 68: Durchmesser
- 70: Durchmesser

## Patentansprüche

1. Verfahren zur Bestimmung des Füllvolumens eines mit einer Flüssigkeit (30) zumindest teilweise gefüllten, verschlossenen Behälters (16), welcher mit elektromagnetischer Strahlung (26) beaufschlagt wird, wobei zumindest ein Teil der vom Behälter transmittierten Strahlung (28) in einer Bildebene ortsaufgelöst detektiert und einer Auswerteeinheit (22) zugeführt wird, **dadurch gekennzeichnet, dass** zur Füllvolumenbestimmung zumindest die Füllstandshöhe (36) der Flüssigkeit (30) im Behälter (16) und zumindest eine Innenquerschnittsfläche des Behälters (16) aus den detektierten Bilddaten ermittelt werden.

2. Verfahren nach Anspruch 1, wobei der Behälter (16) zumindest abschnittsweise eine zylindrische Geometrie aufweist und zur Ermittlung der Innenquerschnittsfläche des Behälters (16) der Innendurchmesser oder der Außendurchmesser (48) und die Wanddicke des Behälters (16) aus den detektierten Bilddaten ermittelt werden.

3. Verfahren nach Anspruch 2, wobei zur Ermittlung der Wanddicke des Behälters (16) ein objektseitiger und/oder bildseitiger telezentrischer Strahlengang zwischen Strahlungsquelle (12) und Detektor (20) vorgesehen ist und wobei der Innendurchmesser und/oder die Wanddicke aus den Abständen detektierter Intensitätsmaxima (56, 58) von durch die Behälterwand (50, 52) transmittierter Strahlung (56, 58) ermittelt wird.

4. Verfahren nach einem der vorhergehenden Ansprüche, wobei die Füllstandshöhe (36) anhand der Positionen eines oberen (46) und eines unteren Flüssigkeitsspiegels (44) aus den detektierten Bilddaten ermittelt wird.

5. Verfahren nach einem der vorhergehenden Ansprüche, wobei zur Ermittlung des Außendurchmessers (48) des Behälters und/oder zur Ermittlung der Positionen der Flüssigkeitsspiegel (44, 46) der Behälter diffus beleuchtet oder durchleuchtet und die Flüssigkeitsspiegel auf dem Detektor abgebildet werden.

6. Verfahren nach einem der vorhergehenden Ansprüche 4 oder 5, wobei zur Positionsbestimmung zumindest eines Flüssigkeitsspiegels (44, 46) die Intensitäten benachbarter Bildpunkte zeilen- und/oder spaltenweise untereinander und/oder mit einem vorgegebenen Schwellwert verglichen werden.

7. Verfahren nach einem der vorhergehenden Ansprüche 3 bis 6, wobei für die Zuordnung von einer in der Bildebene vorhandenen Anzahl an Pixel zu einem tatsächlichen Längenmaß, der Pixelwert eines mittels diffuser Beleuchtung ermittelten Außendurchmessers (48) des Behälters mit dem mittels telezentrischer Beleuchtung ermittelten Pixelwert verglichen wird.

8. Verfahren nach einem der vorhergehenden Ansprüche 4 bis 7, wobei die Außenkonturen (38, 40) eines sich in der Bildebene oval darstellenden Flüssigkeitsspiegels mittels Intensitätsvergleich benachbarter Bildpunkte bestimmt werden und zwischen den gekrümmten Außenkonturen eine geradlinige geometrische Mittellinie als Flüssigkeitsspiegel (44) ermittelt wird.

9. Verfahren nach einem der vorhergehenden Ansprüche, wobei zur Ermittlung des Außendurchmessers (48) des Behälters die radiale Ausdehnung eines das Behälterinnenvolumen begrenzenden Stopfens (34) aus den detektierten Bilddaten ermittelt wird.

10. Verfahren nach einem der vorhergehenden Ansprüche, wobei zur Eliminierung von Gas- insbesondere Luftblasen innerhalb der Flüssigkeit, der Behälter (16) zumindest zeitweise in Rotation versetzt wird, wobei die Rotationsachse (25) senkrecht oder schräg insbesondere senkrecht zur optischen Achse des Detektors (20) bzw. der Strahlungsquelle (12) und/oder parallel zur Zylinderlängsachse verläuft.

11. Verfahren nach einem der vorhergehenden Ansprüche, wobei zumindest eine Querschnittsausdehnung (68, 70) von in der Flüssigkeit eingeschlossenen Gas-, insbesondere Luftblasen (66) aus den detektierten Bilddaten ermittelt, einem entsprechendes Gasblasenvolumen zugeordnet und vom ermittelten Füllvolumen subtrahiert wird.

12. Verfahren nach einem der vorhergehenden Ansprüche, wobei eine Abfolge von n Bilddaten zeitlich versetzt aufgenommen wird, wobei der Behälter zwischen den Einzelaufnahmen um jeweils 360° / n um eine Achse senkrecht zur optischen Achse gedreht wird.

13. Vorrichtung zur Bestimmung des Füllvolumens eines mit einer Flüssigkeit (30) zumindest teilweise gefüllten Behälters (16) mit einer Strahlungsquelle (12) zur Beaufschlagung des Behälter mit elektromagnetischer Strahlung (26) und mit einem mit einer Auswerteeinheit (22) koppelbaren Detektor (20) zur Detektion von durch den Behälter (16) transmittierter und/oder daran reflektierter Strahlung (28) und mit Strahlführungsmitteln (14, 18) zwischen Detektor (20) und Strahlungsquelle (12) zum zumindest abschnittsweisen Abbilden des Behälters (16) auf dem Detektor (20) **dadurch gekennzeichnet, dass** die Auswerteeinheit (22) dazu ausgebildet ist, aus den detektierten Bilddaten eine Füllstandshöhe der Flüssigkeit (30) im Behälter (16) und zumindest eine Innenquerschnittsfläche des Behälters (16) selbsttätig zu ermitteln.

14. Vorrichtung nach Anspruch 13, wobei zur Bestimmung des Innenquerschnitts oder der Wanddicke des Behälters (16) eine Beaufschlagung des Behälter mit zueinander parallelen Strahlen mittels zumindest einem telezentrischen Objektiv (16, 18) zwischen Detektor (20) und Strahlungsquelle (12) vorgesehen ist.

15. Vorrichtung nach einem der vorhergehenden Ansprüche 13 oder 14, wobei ein um eine Drehachse (25) drehbarer Drehteller (24) zur Aufnahme des Behälters (16) vorgesehen ist, wobei die Drehachse (25) des Drehtellers (24) im Wesentlichen parallel oder überlappend zur Zylinderlängsachse verläuft.

16. Computerprogrammprodukt mit Programmmitteln zur Bestimmung des Füllvolumens eines mit einer Flüssigkeit zumindest teilweise gefüllten Behälters, welche dazu ausgebildet sind, aus den von einem Detektor zur Verfügung gestellten Bilddaten eine Füllstandshöhe der Flüssigkeit und einen Innendurchmesser des Behälters nach dem Verfahren gemäß einem der Patentansprüche 1 bis 11 selbsttätig zu ermitteln und zur Bestimmung des Füllstandsvolumens miteinander zu multiplizieren.
